# DEMANDE DE BREVET EUROPEEN

(11) **EP 4 700 066 A1**
(43) Date de publication de la demande: **25.02.2026**
(21) Numéro de dépôt: 25193337.0
(22) Date de dépôt: 01.08.2025
(51) Int. Cl.: C08G 69/46, C07D 201/12, C07C 209/62, C07C 211/12, C08J 11/10

(54) **PROCÉDÉ D'AMORPHISATION D'UN MATÉRIAU COMPRENANT UN POLYMÈRE**

(30) Priorité: 01.08.2024 FR 2408551
(71) Demandeur: Renault s.a.s, 92100 Boulogne Billancourt (FR); Centre National de la Recherche Scientifique, 75016 Paris (FR)
(72) Inventeur: COLIN, Annie, 75005 PARIS (FR); CRETON, Costantino, 75005 PARIS (FR); LAY, Edwige, 78280 GUYANCOURT (FR); FURTWENGLER, Pierre, 78284 GUYANCOURT (FR); GERVAT, Laurent, 78284 GUYANCOURT CEDEX (FR)
(74) Mandataire: Casalonga

(57) **Abrégé**

La présente invention concerne un procédé d'amorphisation d'un matériau comprenant au moins un polymère comprenant les étapes suivantes :
a) ajouter le matériau dans une solution S1 contenant au moins un sel et présentant un pH inférieur ou égal à 7 ;
b) exposer la solution S1 à une température T1 supérieure ou égale à 20°C ;
c) maintenir le matériau dans ladite solution S1 jusqu'à l'amorphisation du polymère ;
d) ajuster le pH de la solution S1 à un pH supérieur ou égal à 4 ou transférer le matériau obtenu dans une solution S2 contenant au moins un sel et présentant un pH supérieur ou égal à 4 et dans laquelle la concentration massique en sel est supérieure ou égale à 10% en poids par rapport au poids total de la solution S2.

## Description

### Domaine technique

La présente invention concerne le domaine des matériaux comprenant au moins un polymère. Plus particulièrement, la présente invention concerne un procédé d'amorphisation d'un matériau comprenant au moins un polymère.

### Techniques antérieures

Dans le contexte climatique actuel, de nombreux moyens sont mis en œuvre pour améliorer la durabilité et la recyclabilité des matériaux, par exemple les matières plastiques courantes. Cette problématique environnementale s'insère dans une logique de recherche de réduction des émissions de dioxyde de carbone. L'utilisation de matériaux recyclés est en effet l'un des moyens pour y parvenir. C'est pourquoi des exigences environnementales en vigueur ou à venir en particulier dans le monde de l'automobile imposent un taux de matière recyclée « PCR » (« Post Consumer » en anglais) minimal à incorporer dans les véhicules neufs ainsi que l'intégration de matériaux issus du recyclage de véhicules en fin de vie.

Ainsi, les matières concernées sont les polymères de manière générale, et notamment les polyamides (PA).

Les polyamides (PA) sont une famille de polymères thermoplastiques contenant des fonctions amide et sont généralement semi-cristallins. Ils peuvent être synthétisés soit par polycondensation de diacides et de diamines soit par ouverture de chaînes de lactames. Ils sont nommés grâce au nombre d'atomes de carbone présents dans la chaîne carbonée des monomères pour les homopolymères et au nombre de carbone de l'amine puis de l'acide pour les copolymères.

Les polyamides sont généralement à la fois résistants et ductiles : les liaisons hydrogènes des groupes amides sont à l'origine de la rigidité, résistance aux composés organiques et du haut point de fusion des PA tandis que les parties aliphatiques influence la dureté et la résistance à l'eau. La combinaison exacte de propriétés dépend du type de polyamide.

Une importante variété de polyamide existe commercialement : on peut citer les polyamides 6, 6.6, 4.6, 4.10, 6.10, 6.12, 11, 12, etc. Ils trouvent des applications très répandues dans l'industrie du textile et du transport dont l'automobile, mais aussi dans le bâtiment, la tapisserie, les ustensiles de cuisine, la machinerie, les membranes de désalinisation, etc.

Les polyamides issus du recyclage sont souvent fortement dégradés. Par conséquent, leur recyclage par voie mécanique ne permet pas d'obtenir des qualités suffisantes pour pouvoir être réutilisés dans les industries exigeantes comme l'automobile. Par conséquent, un procédé de dépolymérisation à l'issue duquel une repolymérisation peut être engagée et qui permet d'obtenir des qualités identiques aux matières vierges revêt un fort intérêt.

Diverses voies de dépolymérisation des polyamides ont été étudiées. Néanmoins, le caractère semi-cristallin des polyamides représente un inconvénient pour la dépolymérisation. En effet, la présence de cristallites gêne la mobilité des catalyseurs. Par conséquent, des dégradations superficielles ont été observées, mais aucune dégradation en masse n'a été mise en évidence. Ainsi, les différentes voies étudiées impliquent l'utilisation de conditions dures pour une dépolymérisation efficace puisque des acides forts sont généralement employés et/ou un chauffage à haute température, au-dessus de la température de fusion du matériau, est appliqué. Ces différentes voies sont donc coûteuses d'un point de vue énergétique.

Il serait donc souhaitable de trouver une voie alternative pour à terme envisager des dépolymérisations de polymères, en particulier de polyamides, moins coûteuses, par exemple par bio-dégradation, notamment par voies enzymatiques.

### Exposé de l'invention

L'invention a donc pour objet un procédé d'amorphisation d'un matériau comprenant au moins un polymère comprenant les étapes suivantes :
a) ajouter le matériau dans une solution S1 contenant au moins un sel et présentant un pH inférieur ou égal à 7 ;
b) exposer la solution S1 à une température T1 supérieure ou égale à 20°C ;
c) maintenir le matériau dans ladite solution S1 jusqu'à l'amorphisation du polymère ;
d) ajuster le pH de la solution S1 à un pH supérieur ou égal à 4 ou transférer le matériau obtenu dans une solution S2 contenant au moins un sel et présentant un pH supérieur ou égal à 4 et dans laquelle la concentration massique en sel est supérieure ou égale à 10% en poids par rapport au poids total de la solution S2.

Le procédé d'amorphisation d'un matériau comprenant au moins un polymère selon l'invention permet non seulement de rendre le polymère contenu dans le matériau amorphe mais aussi de stabiliser le caractère amorphe du polymère. Par conséquent, le procédé d'amorphisation représente un préalable extrêmement avantageux dans la mesure où il permet de lever des verrous technologiques forts ouvrant des perspectives pour la dépolymérisation des matériaux comprenant un polymère, notamment du polyamide. En effet, le matériau obtenu à l'issue du procédé d'amorphisation selon l'invention peut être engagé dans des voies de dépolymérisation plus douces et meilleures pour l'environnement que celles actuellement utilisées, par exemple par dégradation enzymatique.

D'autres avantages et caractéristiques de l'invention apparaîtront plus clairement à l'examen de la description détaillée et des dessins annexés sur lesquels :
[Fig 1] est un graphe représentant l'évolution de la prise de masse d'un échantillon d'un matériau de polyamide 6.6 en fonction du temps d'immersion dans différentes solutions salines ;
[Fig 2] est un graphe représentant l'évolution de la prise de masse d'un échantillon d'un matériau de polyamide 6.6 en fonction du temps d'immersion dans une solution de ZnCl₂ ;
[Fig 3] est un graphe représentant l'évolution de la prise de masse d'un échantillon d'un matériau de polyamide 6.6 en fonction du temps d'immersion dans une solution de ZnSO₄ ;
[Fig 4] est un spectre DRX de différents échantillons d'un matériau de polyamide 6.6 après immersion dans une solution de ZnCl₂ ;
[Fig 5] est un spectre DRX de différents échantillons d'un matériau de polyamide 6.6 obtenus à l'issue d'un procédé d'amorphisation selon l'invention ou d'un procédé comparatif.

Il est précisé que l'expression « de... à... » utilisée dans la présente description de l'invention doit s'entendre comme incluant chacune des bornes mentionnées.

Comme indiqué ci-avant, le procédé d'amorphisation d'un matériau comprenant au moins un polymère selon l'invention comprend :
a) ajouter le matériau dans une solution S1 contenant au moins un sel et présentant un pH inférieur ou égal à 7.

Avantageusement, le polymère est un polyamide, de préférence choisi parmi le polyamide 6, le polyamide 6-6 et leur mélange.

Selon un mode de réalisation particulier, le matériau comprenant au moins un polyamide peut être toute pièce en polyamide, par exemple toute pièce en polyamide 6, polyamide 6-6, mais aussi tous matériaux comprenant du polyamide et pouvant contenir une charge, telle des fibres de verre, des charges minérales, telles que du talc, de la poussière minérale, de la montmorillonite, des fibres de carbone, des billes de verres pleines, des billes de verre creuses, et/ou encore une charge inorganique et/ou un nodule organique.

Le matériau comprenant au moins un polyamide peut être une pièce comprenant deux polyamides de nature différente, par exemple une pièce obtenue après mélange de PA 6.6 avec du PA 6 ou inversement. Ce matériau initial est avantageux puisqu'avec une transamidification entre les polyamides en présence, la cristallinité du mélange résultant est d'autant plus diminuée. La pièce peut être récupérée par micro-granulation avant d'être engagée dans le procédé selon l'invention.

Le matériau comprenant au moins un polymère, en particulier un polyamide, peut se présenter sous toute forme.

Avantageusement, le matériau est une pièce présentant un rapport surface/volume important. Un tel rapport surface/volume peut être obtenu après broyage de la pièce avant qu'elle ne soit engagée dans le procédé selon l'invention. Ainsi, le matériau comprenant au moins un polymère peut être une pièce broyée.

Une autre forme dudit matériau peut être un film pouvant présenter une épaisseur allant de 50 à 500 µm.

Avantageusement, le sel de la solution S1 est un sel choisi parmi CuCl₂, ZnBr₂, ZnCl₂ et leurs mélanges.

De manière particulièrement préférée, le sel de la solution S1 est le sel ZnCl₂.

De manière particulièrement préférée, lorsque le sel est le sel ZnCl₂, le pH de la solution S1 est avantageusement inférieur ou égal à 2.

Avantageusement, la concentration massique en cation du sel est supérieure ou égale à 20% en poids, de préférence va de 20 à 85% en poids par rapport au poids total de la solution S1.

Avantageusement, la concentration massique en sel est supérieure ou égale à 20% en poids, de préférence va de 20 à 85% en poids par rapport au poids total de la solution S1.

Plus préférentiellement, la concentration massique du sel de zinc, de préférence du ZnCl₂, est supérieure ou égale 20% en poids par rapport au poids total de la solution S1.

Encore plus préférentiellement, la concentration massique du sel de zinc, de préférence du ZnCl₂, va de 40 à 85% en poids, mieux de 70 à 85% en poids par rapport au poids total de la solution S1.

Avantageusement, la solution S1 contenant au moins un sel est une solution saturée. L'emploi d'une solution S1 saturée est avantageuse dans la mesure où le temps d'amorphisation est rapide. Néanmoins, toute concentration massique peut être utilisée mais le temps d'amorphisation sera plus long. L'homme du métier sait ajuster la concentration massique d'une telle solution.

Avantageusement, le pH de la solution S1 est inférieur ou égal à 2, de préférence inférieur ou égal à 1. Plus préférentiellement, le pH de la solution S1 est préférentiellement égal à 0. On pourra le cas échéant avantageusement ajuster le pH de la solution S1 en ajoutant un acide à la solution.

Avantageusement, la solution S1 comprend un solvant pouvant être de l'eau. Selon un mode de réalisation, le solvant peut être de l'eau en combinaison avec au moins un autre solvant différent de l'eau. Le solvant différent de l'eau peut être choisi parmi les alcools, tels que l'éthanol, le méthanol, le propanol, le butanol ; l'acide acétique ; l'acide chloridrique ; l'acide sulfurique ; le DMSO ; le THF ; la pyridine, etc.

Ainsi, selon un mode de réalisation, la solution S1 comprend un mélange de solvants, par exemple de l'eau et au moins un autre solvant différent de l'eau. En particulier, le mélange de solvants peut être un mélange de 2 solvants, 3 solvants, 4 solvants, etc.

Comme indiqué précédemment, le procédé selon l'invention comprend :
b) exposer la solution S1 à une température T1 supérieure ou égale à 20°C ;
c) maintenir le matériau dans ladite solution S1 jusqu'à l'amorphisation du polymère.

Avantageusement, les deux étapes b) et c) sont simultanées.

Ainsi, le matériau est maintenu dans la solution S1, avec une exposition à une température T1 supérieure ou égale à 20°C, jusqu'à l'amorphisation du polymère, en particulier du polyamide.

Le matériau est ainsi maintenu dans la solution S1 pendant une durée nécessaire pour obtenir le polymère amorphisé, en particulier le polyamide amorphisé. Cette durée dépend de la température T1. La durée nécessaire pour obtenir le polymère amorphisé, en particulier le polyamide amorphisé, sera plus longue si la température T1 est égale à 20°C que si cette température T1 est plus élevée, par exemple à 50°C ou encore à 80°C, à pression atmosphérique.

Par exemple, pour une température de 20°C, la durée pour obtenir le polymère amorphisé, en particulier le polyamide amorphisé, peut être de plusieurs semaines.

Pour une température T1 égale à 80°C, à pression atmosphérique, la durée nécessaire pour obtenir le polymère amorphisé, en particulier le polyamide amorphisé peut aller de quelques minutes à quelques jours.

Plus la température est élevée, plus l'amorphisation est rapide. Ainsi, l'utilisation d'une température la plus élevée possible est avantageuse.

Avantageusement, à pression atmosphérique, la température T1 peut aller de 20°C à 80°C, de préférence de 50 à 80°C, plus préférentiellement de 70 à 80°C, de manière particulièrement préférée, la température T1 est égale à 80°C.

Selon un autre mode de réalisation, le matériau peut être maintenu dans la solution S1 dans un environnement sous pression. Avantageusement la pression maximale pouvant être appliquée est celle à laquelle le solvant change d'état, tout en tenant compte de la température de fusion du polymère. Par exemple, dans le cas de l'eau en tant que solvant et le polyamide 6.6 en tant que polymère, la température de fusion du polyamide est de 260°C, le matériau peut alors maintenu à une pression strictement supérieure à 1000 hPa et inférieure ou égale à 50 000 hPa.

Avantageusement, lorsque le matériau est maintenu dans la solution S1 sous pression, la température T1 maximale pouvant être appliquée est celle à laquelle une ébullition apparaît. Ceci peut être déterminé à la lecture des diagrammes de phase des solvants connus par l'homme du métier.

Selon un mode de réalisation préféré, lorsque le matériau est maintenu dans la solution S1 sous pression, la température T1 peut aller de 20°C à la température de fusion du polymère, notamment de 20 à 260°C.

Lorsque la température T1 est supérieure à 20°C, la solution S1, dans laquelle se trouve le matériau comprenant au moins un polymère, en particulier un polyamide, peut être chauffée par tout moyen connu de l'homme du métier, par exemple à l'aide d'une étuve, d'une paroi, d'un collier chauffant, etc.

Avantageusement, la température T1 est supérieure ou égale à la température de transition vitreuse du polymère, en particulier du polyamide.

La température de transition vitreuse du polymère, en particulier du polyamide, peut être déterminée par toute méthode connue de l'homme du métier. En particulier, elle peut être mesurée de manière connue par DSC (« Differential Scanning Calorimetry » en anglais), au second passage, par exemple et sauf indications différentes spécifiées dans la présente demande, selon la norme ISO 11357 - 2018 (appareil DSC "822-2" de Mettler Toledo ; atmosphère azote). Elle peut être également mesurée selon la méthode suivante : échantillons préalablement portés de -50°C à 285°C (10°C à 20°C/min), puis refroidis rapidement jusqu'à -50°C, avant enregistrement final de la courbe de DSC de -50°C à 285°C, selon une rampe de 10°C à 20°C/min).

Comme indiqué précédemment, le matériau est maintenu dans ladite solution S1 jusqu'à l'amorphisation du polymère, en particulier du polyamide.

L'homme du métier sait déterminer lorsque le polymère, en particulier le polyamide, est amorphisé.

L'amorphisation du polymère, en particulier du polyamide, peut être suivie tout au long du processus par des mesures bien connues de l'homme du métier, par exemple par DSC ou par des mesures de diffractions de rayons X. Les mesures de DSC peuvent être réalisées par toute méthode connue de l'homme du métier, par exemple selon la méthode décrite ci-avant. Les mesures de diffractions de rayons X peuvent être réalisées par toute méthode connue de l'homme du métier, par exemple selon la méthode décrite ci-dessous.

Ainsi, tout au long du processus, des prélèvements peuvent être effectués à intervalles réguliers et le taux de cristallinité peut ainsi être déterminé.

Avantageusement, le polymère, en particulier le polyamide, est considéré comme suffisamment amorphisé lorsque le taux de cristallinité est inférieur à 5% de préférence inférieur à 1%.

Dans le cadre de la présente invention, il peut être considéré que le polymère, en particulier le polyamide, est suffisamment amorphisé lorsqu'il est dans des conditions compatibles pour envisager une dépolymérisation par bio-dégradation, notamment par dégradations enzymatiques.

Comme précisé ci-avant, le procédé selon l'invention comprend également : d) ajuster le pH de la solution S1 à un pH supérieur ou égal à 4 ou transférer le matériau obtenu dans une solution S2 contenant au moins un sel et présentant un pH supérieur ou égal à 4 et dans laquelle la concentration massique en sel est supérieure ou égale à 10% en poids par rapport au poids total de la solution S2.

Ainsi, à l'issue de l'étape c), le pH de la solution S1 est ajusté jusqu'à un pH supérieur ou égal à 4. Alternativement, le matériau obtenu peut être transféré dans une solution S2 telle qu'indiquée ci-avant.

Avantageusement, lorsque le pH de la solution S1 est strictement inférieur à 4, lors de l'étape a), de préférence inférieur ou égal à 2, alors, lors de l'étape d), le pH de la solution S1 est ajusté à la hausse jusqu'à un pH supérieur ou égal à 4.

Cette étape d) est particulièrement avantageuse car elle permet de stabiliser le caractère amorphe du polymère, en particulier du polyamide, i.e. de le conserver, ce qui est essentiel pour la présente invention.

Avantageusement, lors de l'étape d), le pH de la solution S1 va de 4 à 5, de préférence de 4 à 4,5.

Le pH de la solution peut être ajusté par tout moyen connu de l'homme du métier, par exemple par ajout de NaOH.

Selon un mode de réalisation préféré, le sel de la solution S2 est un sel choisi parmi CuCl₂, ZnBr₂, ZnCl₂ et leurs mélanges, de manière particulièrement préférée le sel de la solution S2 est le sel ZnCl₂.

Avantageusement, le pH de la solution S2 va de 4 à 5, de préférence de 4 à 4,5.

Avantageusement, la concentration massique en sel va de 10 à 85% en poids, de préférence de 20 à 85% en poids par rapport au poids total de la solution S2.

Plus préférentiellement, la concentration massique en sel de zinc, de préférence en ZnCl₂, va de 10 à 85% en poids, encore plus préférentiellement de 20 à 85% en poids, mieux de 40 à 85% en poids, mieux encore de 70 à 85% en poids par rapport au poids total de la solution S2.

De manière préférée, lors de l'étape d), la solution S1, ou la solution S2, est chauffée à une température allant de 40 à 60°C.

Le procédé selon l'invention peut comprendre en outre, avant l'étape a), un chauffage du matériau, de préférence à une température supérieure ou égale à 50°C.

Cette étape préalable à l'étape a) permet de sécher de sécher le matériau comprenant au moins un polymère, en particulier un polyamide. Plus la température est élevée, plus le matériau est séché rapidement. Ainsi, l'utilisation d'une température la plus élevée possible lors de cette étape est avantageuse puisqu'il permet de réduire le temps de séchage.

La température appliquée lors de cette étape peut avantageusement aller de 50 à 110°C, de préférence de 80 à 110°C. A ces températures allant de 80 à 110°C, la durée de séchage peut être typiquement de 4 heures.

A l'issue du procédé selon l'invention, le matériau obtenu est dans des conditions compatibles pour envisager une dépolymérisation par bio-dégradation, notamment par dégradations enzymatiques.

La présente invention est illustrée de manière non-limitative par les exemples suivants.

### Exemples

### Matériel et méthodes de mesure

Des films de polyamide 6.6 (PA 6.6) d'épaisseur 500 µm achetés en rouleaux de 1m*20cm chez la société Goodfellow ont été utilisés (référence AM32-FM-000200). Les films ont été fabriqués par extrusion puis étirage sur calendres métalliques dont l'écartement détermine l'épaisseur du film. Ils ont donc une orientation dans la direction de l'extrusion qui peut facilement être déterminée en chauffant entre la température de transition vitreuse T_{g} et la température Tₘ.

Des échantillons rectangulaires de 1*10 cm ont été découpés aux ciseaux et des échantillons de type ISO527 1BA ont été découpés à l'emporte-pièce. Des échantillons de PA 6.6 de type ISOS27 5A d'épaisseur 2 mm ont également été fournis par la société Celanese (ZYTEL 101L NC010). Les solutions salines aqueuses utilisées, détaillées dans la suite, ont été mélangées à partir de sels achetés chez la société Sigma Aldrich Merck. Les sels étant très hygroscopiques, un soin particulier a été apporté pour leur conservation : les boîtes de sels ont été descellées juste avant la préparation des solutions.

### Mesure de prise de masse

Les conditions initiales sont définies après séchage de l'échantillon à 80°C en étuve ventilée pendant au minimum 24h. La masse de l'échantillon vaut alors W₀. L'échantillon est alors immergé dans un bain fermé de solution saline à 50°C placé dans une étuve ventilée. Aucune contrainte mécanique n'a été appliquée. L'échantillon est régulièrement sorti du bain, rincé à l'eau distillée, essuyé et pesé (Wt) avec une précision de 0,1 mg. L'évolution du % prise de masse définie comme suit :
% prise de masse = (100*(Wₜ-W₀))/W₀.

Les mesures de prise de masse ont été arrêtées lorsque la courbe a atteint un plateau bien défini, après quelques centaines d'heures d'immersion.

### Diffraction de rayons X (DRX)

Des carrés d'échantillons adaptés au support ont été découpés dans un film de PA 6.6 de 500 µm d'épaisseur. Ils ont été soumis aux diverses conditions décrites précédemment et ci-après puis collés au support et équilibrés à température ambiante. Le spectre DRX a été acquis pendant 1h sur un diffractomètre « Philips X'Pert », sous une tension de 40kV et une intensité de 40mA, pour des angles 2θ entre 10 et 60°.

### Exemple 1

Des échantillons de PA 6.6 de type ISO527 5A présentant une épaisseur de 2 mm ont été ajoutés dans différentes solutions salines, les solutions étant chauffées à 50°C en étude ventilée.

Des solutions salines aqueuses de NaCl, CaCl₂, ZnCl₂ et ZnSO₄ ont été mises en œuvre, en particulier :
- trois solutions de NaCl présentant une concentration massique de 4% (courbe B), 13,5% (courbe C) et 27% (courbe D) en poids, respectivement, par rapport au poids total de la solution ;
- trois solutions de CaCl₂ présentant une concentration massique de 8,7% (courbe E), 29% (courbe F) et 58% (courbe G) en poids, respectivement, par rapport au poids total de la solution ;
- trois solutions de ZnCl₂ présentant une concentration massique de 12% (courbe H), 41% (courbe I) et 82% (courbe J) en poids, respectivement, par rapport au poids total de la solution ; et
- trois solutions de ZnSO₄ présentant une concentration massique de 6,3% (courbe M), 21% (courbe L) et 42% (courbe K) en poids, respectivement, par rapport au poids total de la solution.

Par ailleurs, un échantillon de PA 6.6 de type ISO527 5A a été ajouté dans de l'eau pour servir de référence (courbe A).

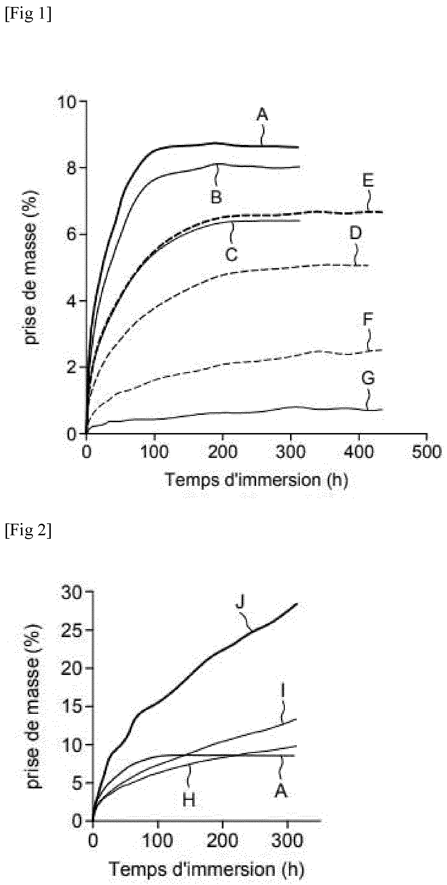

L'évolution de la prise de masse en fonction du temps d'immersion a été suivie, comme cela peut être observé sur les figures 1 à 3.

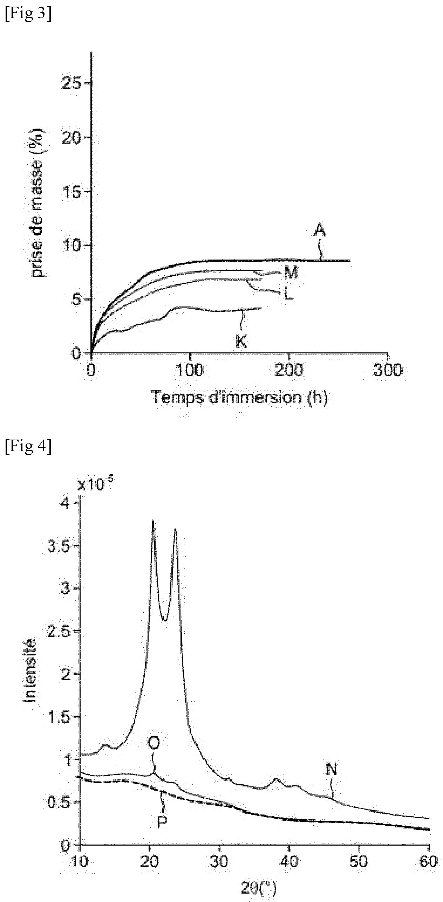

Comme on peut le voir sur les figures 1 et 3, une prise de masse est observée en fonction du temps avec une augmentation de la masse jusqu'à atteindre un plateau (cf courbes B à G et courbes K à M).

En revanche, le comportement est différent pour les échantillons plongés dans les solutions salines de ZnCl₂. En effet, comme on peut le voir sur la figure 2, une augmentation de la masse est observée mais aucun plateau n'a été atteint. La figure 2 montre des temps d'immersion jusqu'à un peu plus de 300 heures, mais des mesures complémentaires ont été effectuées avec des temps d'immersion jusqu'à des milliers d'heures. Même après ces temps d'immersion, aucun plateau n'a été observé.

Ainsi, ces figures démontrent clairement que non seulement le sel de zinc mais aussi les ions chlorures ont un impact sur la prise de masse. Ainsi, il est démontré que le sel ZnCl₂ a un impact.

Des mesures ont également été faites pour des échantillons de PA 6.6 de type ISO527 5A présentant une épaisseur de 2 mm et qui ont été ajoutés dans différentes solutions salines, les solutions étant chauffées non pas à 50°C en étude ventilée mais à 25°C en étuve ventilée.

Les observations ci-dessus sont également valables pour les « essais à 25°C ».

### Exemple 2

Dans cet exemple, un carré d'échantillon adapté au support a été découpé dans un film de PA 6.6 présentant une épaisseur de 500 µm, puis après un chauffage des échantillons à une température de 80°C en étuve ventilée pendant 48 heures, l'échantillon a été ajouté dans différentes solutions salines aqueuses, les solutions étant chauffées à 50°C en étude ventilée.

Des solutions salines aqueuses de NaCl, CaCl₂, ZnCl₂ et ZnSO₄ ont été mises en œuvre, en particulier :
- une solution de NaCl présentant une concentration massique de 27% en poids par rapport au poids total de la solution ;
- une solution de CaCl₂ présentant une concentration massique de 57% en poids par rapport au poids total de la solution ;
- une solution de ZnSO₄ présentant une concentration massique de 42% en poids par rapport au poids total de la solution ; et
- une solution de ZnCl₂ présentant une concentration massique de 82% en poids par rapport au poids total de la solution (présentant un pH de 0) ; et

Par ailleurs, un carré d'échantillon découpé dans un film de PA 6.6 présentant une épaisseur de 500 µm a été chauffé à une température de 80°C en étuve ventilée pendant 48 heures. Cet échantillon est utilisé pour servir de référence (courbe N).

Puis, les spectres DRX ont été acquis dans les conditions précédemment décrites.

Comme on peut le voir sur la figure 4, trois courbes sont présentes :
- la courbe N correspond à l'échantillon référence ;
- la courbe O correspondant à l'échantillon qui a été immergé dans une solution de ZnCl₂ telle que décrite ci-dessus pendant une durée de 24 heures ;
- la courbe P correspondant à l'échantillon qui a été immergé dans une solution de ZnCl₂ telle que décrite ci-dessus pendant une durée de 48 heures.

Il s'agit du même échantillon tout au long de l'expérience avec le temps d'immersion qui varie.

Il apparaît clairement que le caractère cristallin du PA 6.6 a disparu, en observant les courbes O et P. Cela signifie qu'une amorphisation du PA 6.6 a été réalisée grâce à la solution de ZnCl₂.

Les spectres DRX des échantillons qui ont été immergés dans les autres solutions salines (NaCl, CaCl₂ et ZnSO₄) ne sont pas représentés sur la figure 4 car ils sont similaires à celui de l'échantillon de référence, et donc les courbes obtenues sont similaires à celle de la courbe N.

Ainsi, la figure 4 démontre clairement que le sel ZnCl₂ permet d'amorphiser le PA 6.6.

### Exemple 3

Dans cet exemple, on reprend la procédure de l'exemple 2 mais on remplace le PA 6.6 par du PA 6. On constate au bout du même temps la même transformation démontrant ainsi l'amorphisation.

### Exemple 4

Dans cet exemple, les conditions de l'exemple 2 telles que décrites ont été reprises.

Puis, le pH de la solution comprenant l'échantillon a été ajusté à un pH égal à 4, par ajout de NaOH, la solution étant chauffée à 50°C en étude ventilée, avec différents temps d'immersion. En fin d'expérience, la solution a été filtrée.

Un autre échantillon qui a été préalablement ajouté (et donc immergé) dans une solution de ZnCl₂ présentant une concentration massique de 82% en poids par rapport au poids total de la solution (présentant un pH de 0), a été lavé en le plongeant dans de l'eau.

Par conséquent, l'échantillon, qui est immergé dans la solution dont le pH a été ajusté à un pH égal à 4, est un échantillon obtenu à l'issue d'un procédé selon l'invention.

L'échantillon qui a été lavé, a été obtenu à l'issue d'un procédé comparatif.

Puis, les spectres DRX ont été acquis dans les conditions précédemment décrites.

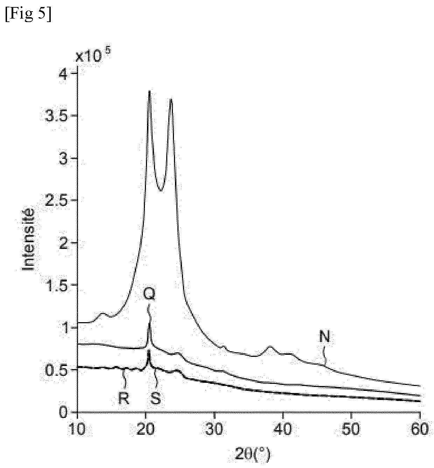

Comme on peut le voir sur la figure 5, quatre courbes sont présentes :
- la courbe N correspond à l'échantillon référence ;
- la courbe Q correspondant à l'échantillon qui a été immergé dans une solution de ZnCl₂ telle que décrite ci-dessus pendant une durée de 24 heures ;
- la courbe R correspondant à l'échantillon qui a été immergé dans une solution de ZnCl₂ telle que décrite ci-dessus pendant une durée de 48 heures ;
- la courbe S correspondant à l'échantillon qui a été immergé dans une solution de ZnCl₂ telle que décrite ci-dessus pendant une durée de 336 heures.

Il s'agit du même échantillon tout au long de l'expérience avec le temps d'immersion qui varie.

Il apparaît clairement que le caractère cristallin du PA 6.6 n'est pas réapparu après observation des courbes Q à S. Une très légère recristallisation (< 1%) peut cependant être noté. Cela signifie que l'amorphisation du PA 6.6 a été stabilisée grâce à la solution de ZnCl₂ présentant un pH plus élevé suite à l'ajustement du pH.

Une recristallisation de l'échantillon qui a été lavé, i.e. l'échantillon obtenu à l'issue du procédé comparatif, a été constatée par mesure de DSC. Cela signifie que le caractère amorphe du polyamide n'a pas été stabilisé.

Ainsi, sans l'étape d) du procédé selon l'invention, un simple lavage, par exemple par une immersion dans de l'eau, du matériau obtenu conduit immédiatement à la recristallisation du matériau, ce qui est à éviter. Le polyamide retrouve alors son caractère semi-cristallin.

Ainsi, la figure 5 démontre clairement que le procédé selon l'invention permet d'amorphiser un matériau comprenant au moins du polyamide mais aussi de stabiliser le caractère amorphe du polyamide.

Par conséquent, le procédé d'amorphisation représente un préalable extrêmement avantageux dans la mesure où il permet de lever des verrous technologiques forts ouvrant des perspectives dans la dégradation des matériaux comprenant un polymère, en particulier un polyamide. En effet, le matériau obtenu à l'issue du procédé d'amorphisation selon l'invention peut être engagé dans des voies de dépolymérisation plus douces que celles actuellement utilisées, notamment des procédés de bio-dégradation, en particulier par voies enzymatiques.

## Revendications

1. Procédé d'amorphisation d'un matériau comprenant au moins un polymère comprenant les étapes suivantes :
a) ajouter le matériau dans une solution S1 contenant au moins un sel et présentant un pH inférieur ou égal à 7 ;
b) exposer la solution S1 à une température T1 supérieure ou égale à 20°C ;
c) maintenir le matériau dans ladite solution S1 jusqu'à l'amorphisation du polymère ;
d) ajuster le pH de la solution S1 à un pH supérieur ou égal à 4 ou transférer le matériau obtenu dans une solution S2 contenant au moins un sel et présentant un pH supérieur ou égal à 4 et dans laquelle la concentration massique en sel est supérieure ou égale à 10% en poids par rapport au poids total de la solution S2.

2. Procédé selon la revendication 1, **caractérisé en ce que** le polymère est un polyamide, de préférence choisi parmi le polyamide 6, le polyamide 6.6 et leur mélange.

3. Procédé selon la revendication 1 ou 2, **caractérisé en ce que** le sel de la solution S1 est un sel choisi parmi CuCl₂, ZnBr₂, ZnCl₂ et leurs mélanges, de manière particulièrement préférée le sel est le sel ZnCl₂.

4. Procédé selon l'une quelconque des revendications précédentes, **caractérisé en ce que** le pH de la solution S1 est inférieur ou égal à 2, de préférence inférieur ou égal à 1.

5. Procédé selon l'une quelconque des revendications précédentes, **caractérisé en ce que** la température T1 est supérieure ou égale à la température de transition vitreuse du polymère.

6. Procédé selon l'une quelconque des revendications précédentes, **caractérisé en ce que**, lors de l'étape d), le pH de la solution S1 va de 4 à 5, de préférence de 4 à 4,5.

7. Procédé selon l'une quelconque des revendications précédentes, **caractérisé en ce que** le sel de la solution S2 est un sel choisi parmi CuCl₂, ZnBr₂, ZnCl₂ et leurs mélanges, de manière particulièrement préférée le sel de la solution S2 est le sel ZnCl₂.

8. Procédé selon l'une quelconque des revendications précédentes, **caractérisé en ce que** le pH de la solution S2 va de 4 à 5, de préférence de 4 à 4,5.

9. Procédé selon l'une quelconque des revendications précédentes, **caractérisé en ce qu'**il comprend en outre, avant l'étape a), un chauffage du matériau, de préférence à une température supérieure ou égale à 50°C.
